# EUROPEAN PATENT SPECIFICATION

(11) **EP 3 925 550 B1**
(45) Date of publication and mention of the grant of the patent: **09.08.2023**
(21) Application number: 20180195.8
(22) Date of filing: 16.06.2020
(51) Int. Cl.: A61B 17/22, A61B 17/225

(54) **A PRESSURE WAVE DEVICE**
DRUCKWELLENVORRICHTUNG
DISPOSITIF D'ONDES DE PRESSION

(43) Date of publication of application: 22.12.2021
(73) Proprietor: Ferton Holding S.A., 2800 Delémont (CH)
(72) Inventor: Evans, Gary, 74200 Allinges (FR); Poulat, Nicolas, 74600 Annecy (FR); Nini, Gaël, 1010 Lausanne (CH)
(74) Representative: Müller Schupfner & Partner Patent- und Rechtsanwaltspartnerschaft mbB

(56) References cited:
- DE-A1-102018 101 215
- US-A1- 2010 198 114

## Description

The present invention refers to pressure wave devices.

Such pressure wave devices are intended to treat humans or animals by using pressure waves, the pressure waves being generated by an impact of an accelerated projectile on an impact body and are well known from the prior art. For example, EP 2 529 792 A1 and EP 2 381 864 B1 describe such pressure wave devices.

Typically, the pressure wave device has a pneumatic drive element for accelerating the projectile. In particular, the pneumatic drive element is intended to accelerate the projectile, which, in turn, hits the impact body, being in contact or close to the surface of the tissue, which should be treated. The pressure wave, generated in the impact body, is transferred into the tissue of a patient by entering the body of the patient via the surface or skin, which is close to the impact body or is in contact with the impact body.

For supplying gas to a handpiece in order to accelerate the projectile, there is, in general, a compressor being part of the pneumatic drive element. Furthermore, the pressure wave device includes a control means for adjusting operating parameters of the pneumatic drive element, preferably a frequency and/or pressure. For example, EP 2 381 864 B1 suggests a compressor, which adapts its power to adjust the strength of the generated pressure wave. Furthermore, it turned out that it is of advantage to use at least two compressors, which can be used to adjust the desired power level of the pressure drive device.

DE 10 2018 101 215 discloses a pressure wave device for treating biological tissue having a handpiece and a pneumatic drive element. The pneumatic drive element has one compressor. US 2010 198 114 A1 relates to a method for setting a pressure application time in an apparatus for treating the human or animal body by mechanical shockwaves, independently of pressure and/or frequency values set, and to a respective apparatus.

However, using several compressors increases a noise level at the same time, which makes the patient feel uncomfortable. Considering the prior art, it is an object of the present invention to provide an improved pressure wave device, in particular having a reduced noise level during operation.

The problem is solved by the pressure wave device according to claim 1, which defines the scope of invention.

Preferred embodiments are incorporated in the dependent claims, the description and the figures. No surgical methods form part of the invention.

According to a first aspect of the present invention, a pressure wave device for treating biological tissue is provided, comprising
- a handpiece,
- a pneumatic drive element, accelerating a projectile of the handpiece, and
- a first compressor and a second compressor to supply gas to the pneumatic drive element for accelerating the projectile,

wherein the pressure wave device is configured to be switchable between
-- a first operation mode, only the first compressor supplying gas to the pneumatic drive element or only the second compressor supplying gas to the pneumatic drive element, and
-- a second operation mode, the first and the second compressor supplying gas to the pneumatic drive element.

In contrast to the prior art, it is possible to switch to a first operation mode, in which only one or a single compressor, namely the first compressor or the second compressor, supplies gas to the pneumatic drive, although two compressors are available. Nevertheless, it is possible to activate at least the second compressor in case of a higher demand of gas. Preferably, the pressure wave device is configured to switch between the first operation mode, only the first compressor supplying gas to the pneumatic drive element or only the second compressor supplying gas to the pneumatic drive element, and a second operation mode, the first compressor and the second compressor supplying gas to the pneumatic drive element. As a result, the noise level being generated by two compressors is limited to specific applications, which need a high amount of gas or a high pressure, which has to be established by the first and second compressor. This turned out as being of advantage, since two compressors working at the same time can cause a huge noise level due to resonance effects, in particular when the engines, driving the compressors, have no precise speed control, which establishes an out-of-phase operation of the first and the second compressor. Therefore, the systems of the prior art, using a first compressor and a second compressor, which operate simultaneously all the time, tend to cause a huge noise level, which is based on an in-phase resonance effect.

The pressure wave device is configured to be switchable between a first operation mode, only the first compressor supplying gas to the pneumatic drive element or only the second compressor supplying gas to the pneumatic drive element, and a second operation mode, the first compressor and the second compressor supplying gas to the pneumatic drive element. As a result, it is possible to operate the pressure wave device, for example on demand, such that only one compressor is operated or at least two compressors are operated.

The pressure wave device comprises a projectile and an impact body, the projectile being accelerated and impacting on the impact body for the generating the pressure wave. In particular, the projectile is accelerated in an inside of a pipe for guiding the projectile and performs a back- and forward-movement, being initiated by the pressurized gas of the pneumatic drive element. Preferably, the pneumatic drive element provides pressurized gas for accelerating the projectile inside the pipe. Furthermore, the pneumatic drive element, in particular, the first compressor and the second compressor are not part of the handpiece, which includes the projectile, the pipe and the impact body, but are incorporated into a base station, being spaced from the handpiece and being connected to the handpiece via a tube. Furthermore, it is conceivable that the handpiece has a regulation unit, as well as an activation switch, wherein the regulation is provided for adjusting the parameters of the pneumatic drive element, in particular a frequency and/or a pressure, and wherein the activation switch triggers the event of a pressure wave. In particular, it is provided that the first compressor and the second compressor are connected in parallel.

During treatment the pressure wave device is located on the skin of a tissue being treated, by placing the impact body on the skin. During the operation the pressure wave generated in impact body enters the skin and travels through the tissue, in particular to the region of interest, being treated by the pressure wave.

The pressure wave devices comprises a control unit for adjusting an amount of requested gas in order to supply gas to the pneumatic drive element. In particular, it is possible to calculate the amount of requested gas, which is needed for realizing the desired parameters of the pressure wave device, from the parameters being set by an operator of the pressure wave device. Based on the level of requested gas or the amount of requested gas the control unit can decide whether the pressure wave device has to be operated in the first operation mode or the second operation mode. In particular, there is a threshold for the amount of requested gas, which can be set by an operator, wherein the amount of requested gas, being set by the control unit, is compared to the threshold value to decide, whether the first operation mode or the second operation mode is used. In particular, if the amount of requested gas exceeds the defined threshold, the pressure wave device automatically switches from the first operation mode to the second operation mode. Consequently, the control unit is preferably configured to start or stop the first and/or second compressor, in particular automatically. Preferably, the pressure wave device is configured to switch from the first operation mode to the second operation mode, when the amount of requested gas, being determined by the control unit, excesses a threshold value.

In particular, it is provided that the control unit switches from the first operation mode to the second operation mode based on the value pair, including a frequency and a pressure for operating the pressure wave device. For example, the pressure wave device is configured to be in the first operation, when
- the requested frequency is smaller than 6 Hz and
- the requested pressure is smaller than 3 bar and the requested frequency is smaller than 9 Hz and/or
- the requested smaller than 2 bar and the requested frequency is lower than 15 Hz and/or
- the requested pressure is smaller than 1 bar and the requested frequency is smaller 16 Hz.

Preferably, a table is used by the control unit to assign the first operation mode to this value pair. In particular, such a table is stored in a storage or a memory unit of the control unit. It is also conceivable that the table, i. e. its assignments, can be updated or changed by the operator. Thus, it is possible to adapt the table to the current specifications of the first compressor and/or the second compressor, in particular when the first compressor and/or the second compressor are exchanged or change their specification during their lifetime. For example, the control unit includes a control loop, which adapts the table to the current specification of the first and/or second compressor from time to time.

The first compressor and/or the second compressor is configured to be operable in different power levels, the power levels being preferably adjusted stepwise. In other words, the first and/or second compressor, in particular its engine or its motor, which drives the first and/or the second compressor, cannot be adjusted continuously. Instead the first and/or second compressor is adjusted stepwise according to set or defined power levels. It turned out that thus the noise level of the whole system of the pressure wave device can be further reduced.

The pneumatic drive element includes a pressure regulation unit, being located downstream to the first compressor and/or the second compressor and upstream to the handpiece. By using a pressure regulation unit it is advantageously possible to continuously adjust the pressure of the supplied gas to a secondary pressurized gas level, being continuously adjustable, although the first compressor and/or the second compressor provide discrete primary pressurized gas levels, based on the discrete power levels of the first compressor and/or second compressor. Preferably, the first compressor and the second compressor use a common pressure regulation unit. For example, such a pressure regulation unit includes a proportional valve and/or magnetic valve for adapting the primary gas pressure to a desired second gas pressure, being used by the pressure wave device to accelerate the projectile, which hits on the impact body. For example, the pressure regulation unit includes a pressure reducer and/or a drain valve to provide a proportional valve.

The pressure wave device is configured to be switchable in the first operation mode between a first sub-mode, only the first compressor supplying gas to the pneumatic drive element, and a second sub-mode, only the second compressor supplying gas to the pneumatic drive element. As a consequence, it is possible to switch between the first compressor and the second compressor during operation or from time to time. As a consequence, the loads to the first compressor and second compressor are reduced over the lifetime of the pneumatic drive element. This has a positive effect on the lifetime of the first and the second compressor, and as a consequence on the lifetime of the pressure wave device.

The first compressor and/or the second compressor is an oil-free-swing piston air compressor. It turned out, that using a pressure wave device being configured to switchable between the first operation mode and the second operation mode has especially huge benefits in case of a first compressor and/or second compressor being an oil-free-swing piston air compressor, in particular regarding the generated noise level, being caused by resonance effects.

Wherever not already described explicitly, individual embodiments or their individual aspects and features can be combined or exchanged with one another without limiting or widening the scope of the described invention, whenever such a combination or exchange is meaningful and in the sense of this invention. Advantages which are described with respect to one embodiment of the present invention are, wherever applicable, also advantageous of other embodiments of the present invention.

In the drawings:
- **Fig. 1**: shows a pressure wave device
- **Fig. 2 and 3**: show circuit diagrams or pressure device according to exemplary embodiments of the present invention

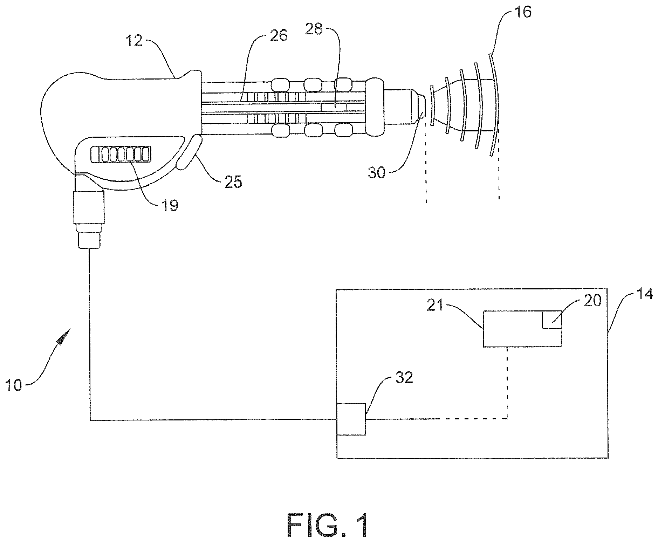

In **figure 1** a pressure wave device 10 according to an exemplary embodiment is shown. Such a pressure wave device 10 is intended to treat tissue of a human body or an animal by generating a pressure wave 16, which acts on the tissue of the body. In particular, it is provided that the pressure wave device 10 is located on the skin of the human or animal body and the pressure wave 16 enters the human or animal body through the skin and acts on tissue inside of the human or animal body. Thereby, the pressure wave device 10 includes an impact body 30, which is in contact with the skin of the human or animal during the treatment. For generating the pressure wave 16, the pressure wave device 10 includes a projectile 28, which is accelerated in a pipe 26 for guiding the projectile 28. The accelerated projectile 28 hits on the impact body 30, generating the pressure wave 16 being emitted by the pressure wave device 10. In particular, the pressure wave device 10 has a handpiece 12, including at least a pipe 26 for accelerating the projectile 28, and a pneumatic drive element 14, which provides pressurized gas to accelerate the projectile 28 inside the pipe 26, in which the projectile 28 is accelerated. Furthermore, it is conceivable that the pressure wave device 10 has a regulation unit 19 and an activation switch 25. By actuating the regulation unit 19 it is possible to define parameters of operation of the pressure wave device 10, whereas the activation switch 25 triggers the generation of the pressure wave 16.

Preferably the pneumatic drive element 14 includes a first compressor 21 being driven by a compressor engine 20. In particular, it is provided that the first compressor 21 is configured to adjust its power level stepwise to establish a certain primary gas pressure being assigned to the first compressor 21. By using a pressure regulation unit 32 it is advantageously possible to adjust the primary gas pressure, being provided by the first compressor 21 by its stepwise power levels, to a secondary gas pressure, which can be adjusted continuously. Thus, it is possible to adjust the parameters of the pressure wave device 10 continuously, although the first compressor 21, in particular the compressor engine 20, is operated by power levels being discrete or stepwise adjustable. It turned out that by using such a combination of first compressor 21 and a pressure regulation unit 32 it is possible to optimize the noise level, generated by the pressure wave device 10, in particular including the pneumatic drive element 14.

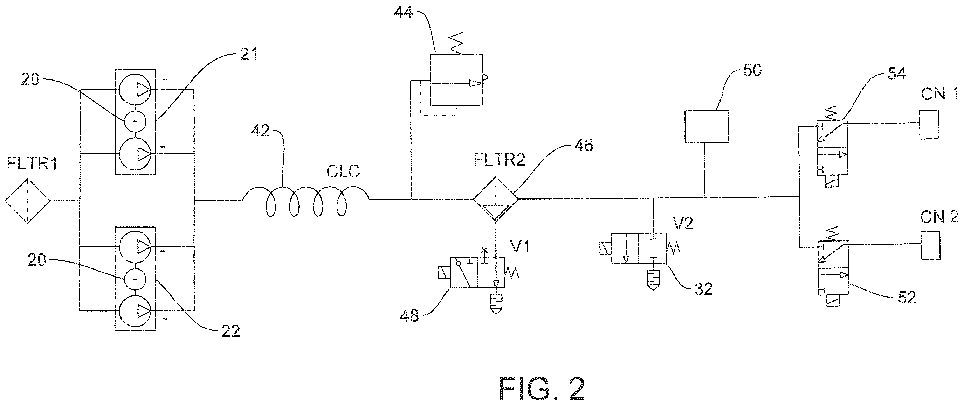

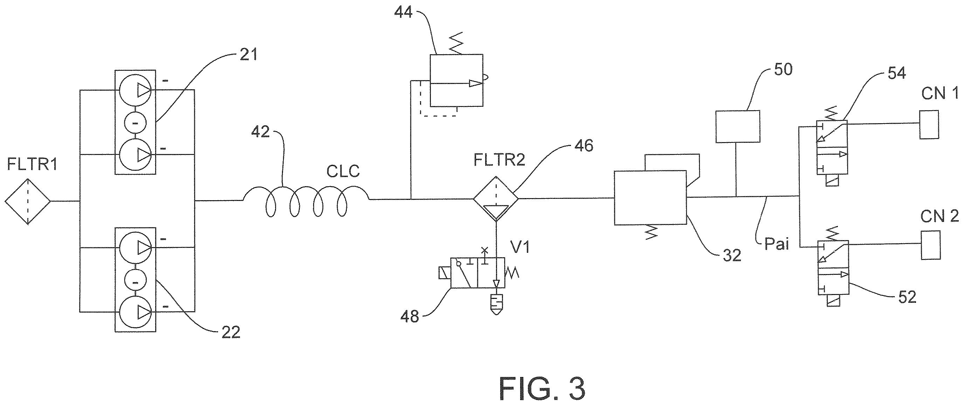

In **figure 2** **and** **figure 3** two circuit diagrams from pressure wave devices 10 according to a first and a second embodiment of the present invention are illustrated. Preferably, it is provided that the pneumatic drive element 14 includes both a first compressor 21 and a second compressor 22 for providing a primary gas pressure. The first compressor 21 and the second compressor 22 are configured such that they can set to discrete power levels, in particular individually. The pressurized gas, being providing by the first compressor 21 and second compressor 22 and having the primary gas pressure, passes a cooling device 42, an overpressure valve 44 and a cooling trap 46 having a vent valve 48 until it reaches the pressure regulating unit 32, being preferably a proportional valve to adjust the pressurized gas from the primary gas pressure level to the desired level, i. e. to the secondary gas pressure level. Preferably, a pressure sensor 50 is located downstream to the pressure regulation unit 32, in order to detect the secondary gas pressure level, which is intended to be used for accelerating the projectile 28 of the pressure wave device 10. In particular, the pressure sensor 50 might be part of a control loop that adjusts the pressure regulation unit 32 to adapt the desired secondary gas pressure level.

Furthermore, the circuit diagram includes a first switch threshold 52 and a second switch threshold 54 being located downstream to the pressure sensor 50. Actually, the embodiments illustrated in the figures 2 and 3 distinguish from each other by the choice of the pressure regulation unit 32. In the embodiment, shown in figure 2, the pressure regulation unit 32 is configured as a valve, in particular as a magnetic valve, preferably configured to withstand pressures up to 7 bars, preferably up to 10 bars. According to the embodiment of figure 3 the pressure regulation unit 32 is a pressure reducer for withstanding pressures up to 7 bars, preferably up to 10 bars.

In particular, it is provided according to the present invention that the pneumatic drive element 14 uses both, the first compressor 21 and the second compressor 22 to provide pressurized gas, in particular having the primary gas pressure level, being adapted subsequently by the pressure regulation unit 32. In particular, it is provided that the pressure wave device 10 is configured to be switchable between
- a first operation mode, only the first compressor 21 supplying gas to the pneumatic drive element 14 or only the second compressor 22 supplying gas to the pneumatic drive element 14, and
- a second operation mode, the first compressor 21 and the second compressor 22 supplying gas to the pneumatic drive element 14.

In other words, the pressure wave device 10 comprises a control unit (not shown) that is configured for switching the pressure wave device 10 from the first operation mode to the second operation mode. Consequently, it is possible to transfer the pneumatic drive element 14 to the second operation only in such cases that huge amount of requested gas is needed to supply the pneumatic drive element 14. As a result, the noise level can be reduced, since by smaller amounts of requested gas, needed to supply the pneumatic drive element 14, only the first compressor 21 or only the second compressor 22 is operated. In particular, it is intended that the pressure wave device 10 is configured to switch from the first operation mode to the second operation mode, when the amount of the requested gas, being adjusted by the regulation unit 19, for example, excesses a threshold value. For example, the control unit identifies the amount of requested gas based on the parameters being adjusted at the handpiece 12. In particular the control unit defines or determines the amount of requested gas by a value pair, including a frequency and a pressure for operating the pressure wave device 10. In other words, the control unit uses a kind of table that is assigned to each pair of frequency or pressure being set on the handpiece 12 of the pressure valve device 10. A corresponding amount of requested gas defines, whether the pressure wave device 10 is operated in the first operation mode or in the second operation mode.

For example, it is conceivable that a requested frequency, being set at the handpiece 12, is smaller than 6 Hz and/or a requested pressure is smaller than 3 bar and the requested frequency is smaller than 9 Hz and/or a requested frequency is smaller than 2 Hz and the requested frequency is smaller than 15 Hz and/or a requested frequency is smaller than 1 Hz and the requested frequency than 10 Hz for operating the pressure wave device in the first operation mode. Preferably, it is provided that the first compressor 21 and the second compressor 22 shares a common pressure regulation unit 32.

Furthermore, it's conceivable that the pressure wave device 10, in particular the control unit is configured to be switchable in the first operation mode between a first sub-mode, only the first compressor 21 supplying gas to the pneumatic drive element 14 and a second sub-mode, only the second compressor 22 supplying to the pneumatic drive element 14. As a consequence, it is possible to switch between the first compressor 21 and second compressor 22 for splitting the work load to both of the compressors 21, 22.

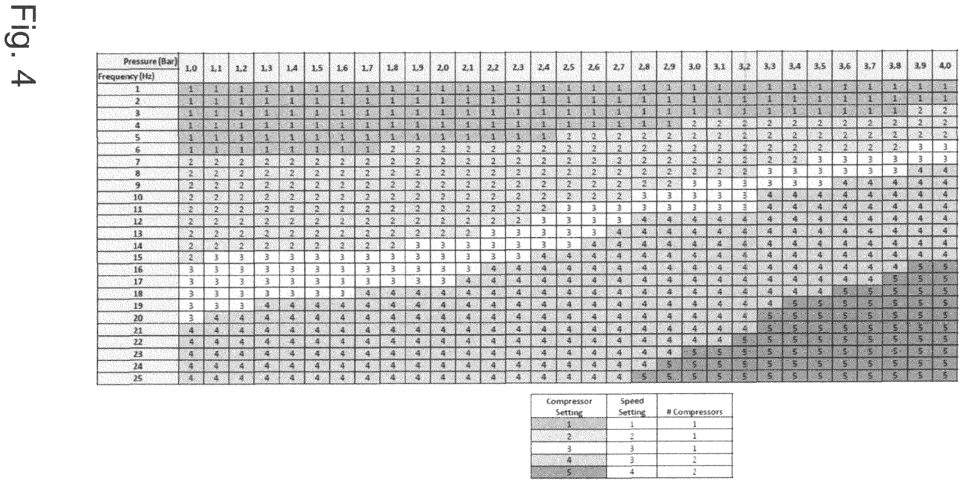

Figure 4 shows an example for a table which is used for setting the first compressor 21 and the second compressor 22, in particular for switching between the first operation mode and the second operation mode. As illustrated the table is formed by the two parameters, frequency and pressure, and defines valve pairs which are assigned to an operation in the first operation mode and an operation in the second operation mode. In particular those value pairs to which the numbers 4 and 5 are assigned, needs an operation in the second operation mode.

### Reference numbers:

- 10: pressure wave device
- 12: handpiece
- 14: pneumatic drive element
- 16: pressure wave
- 19: regulation unit
- 20: compressor engine
- 21: first compressor
- 22: second compressor
- 25: activation switch
- 26: pipe
- 28: projectile
- 30: impact body
- 32: pressure regulation unit
- 42: cooling device
- 44: overpressure valve
- 46: cooling trap
- 48: vent valve
- 50: pressure sensor
- 52: first switch threshold
- 54: second switch threshold

## Claims

1. A pressure wave device (10) for treating biological tissue, comprising
- a handpiece (12),
- a pneumatic drive element (14) accelerating a projectile (28) of the handpiece (12), further comprising
- a first compressor (21) and a second compressor (22) to supply gas to the pneumatic drive element (14) for accelerating the projectile (28),
wherein the pressure wave device (10) is configured to be switchable between
-- a first operation mode, only the first compressor (21) supplying gas to the pneumatic drive element (14) or only the second compressor (22) supplying gas to the pneumatic drive element (14), and
-- a second operation mode, the first compressor (21) and the second compressor (22) supplying gas to the pneumatic drive element (14).

2. The pressure wave device (10) according to claim 1, further comprising a control unit (30) for adjusting an amount of requested gas intended to supply the pneumatic drive element (14).

3. The pressure wave device (10) according to one of the preceding claims, wherein the pressure wave device (10) is configured to switch from the first operation mode to the second operation mode, when the amount of the requested gas, being adjusted via the control unit, excesses a threshold value.

4. The pressure wave device (10) according to one of the preceding claims, wherein the control unit switches from the first operation mode to the second mode based on a value pair, including a frequency and a pressure for operating the pressure valve device (10).

5. The pressure wave device (10) according to claim 4, wherein the pressure wave device (10) is configured to be in the first operation mode, when
- a requested frequency is smaller than 6 Hz and/or
- a requested pressure is smaller than 3 bar and the requested frequency is smaller than 9 Hz and/or
- a requested pressure is smaller than 2 bar and the requested frequency is smaller than 15 Hz and/or
- a requested pressure is smaller than 1 bar and the requested frequency is smaller than 16 Hz.

6. The pressure wave device (10) according to one of the preceding claims, wherein the first compressor (21) and/or the second compressor (22) is configured to be operable in different power levels, the power levels being preferably adjustable stepwise.

7. The pressure wave device (10) according to one of the preceding claims, wherein the pressure wave (10) device includes a pressure regulation unit (32) being located downstream to the first compressor (21) and/or the second compressor (22) and, preferably, upstream to the handpiece (12).

8. The pressure wave device (10) according to claim 7, wherein the pressure regulation unit (32) includes a pressure reducer and/or a drain valve, in particular in form of a proportional valve.

9. The pressure wave device (10) according to one of the preceding claims, wherein the pressure wave device (10) is configured to be switchable in the first operation mode between
-- a first sub-mode, only the first compressor (21) supplying gas to the pneumatic drive element (14) and
-- a second sub-mode, only the second compressor (22) supplying gas to the pneumatic drive element (14).

10. The pressure wave device (10) according to one of the preceding claims, wherein the first compressor (21) and/or the second compressor (22) is an oil free swing piston air compressor.

## Patentansprüche

1. Druckwellengerät (10) zur Behandlung von biologischem Gewebe, umfassend
- ein Handstück (12),
- ein pneumatisches Antriebselement (14), das ein Projektil (28) des Handstücks (12) beschleunigt, ferner umfassend
- einen ersten Kompressor (21) und einen zweiten Kompressor (22) zum Zuführen von Gas zu dem pneumatischen Antriebselement (14) zum Beschleunigen des Projektils (28),
wobei das Druckwellengerät (10) zum Umschalten konfiguriert ist zwischen
- einem ersten Betriebsmodus, wobei nur der erste Kompressor (21) Gas dem pneumatischen Antriebselement (14) zuführt oder nur der zweite Kompressor (22) Gas dem pneumatischen Antriebselement (14) zuführt, und
- einem zweiten Betriebsmodus, wobei der erste Kompressor (21) und der zweite Kompressor (22) dem pneumatischen Antriebselement (14) Gas zuführen.

2. Druckwellengerät (10) nach Anspruch 1, ferner umfassend eine Steuerungseinheit (30) zur Einstellung einer Menge von angefordertem Gas, das zur Versorgung des pneumatischen Antriebselements (14) bestimmt ist.

3. Druckwellengerät (10) nach einem der vorangehenden Ansprüche, wobei das Druckwellengerät (10) konfiguriert ist, um von der ersten Betriebsart in die zweite Betriebsart zu wechseln, wenn die Menge des angeforderten Gases, die über die Steuerungseinheit eingestellt wird, einen Schwellenwert überschreitet.

4. Druckwellengerät (10) nach einem der vorangehenden Ansprüche, wobei die Steuerungseinheit auf Basis eines Wertepaares, das eine Frequenz und einen Druck zum Betreiben des Druckwellengeräts (10) einschließt, von der ersten Betriebsart in die zweite Betriebsart umschaltet.

5. Druckwellengerät (10) nach Anspruch 4, wobei das Druckwellengerät (10) konfiguriert ist, im ersten Betriebsmodus vorzuliegen, wenn
- eine angeforderte Frequenz kleiner als 6 Hz ist und/oder
- ein angeforderter Druck kleiner als 3 bar ist und die angeforderte Frequenz kleiner als 9 Hz ist und/oder
- ein angeforderter Druck kleiner als 2 bar ist und die angeforderte Frequenz kleiner als 15 Hz ist und/oder
- ein angeforderter Druck kleiner als 1 bar ist und die angeforderte Frequenz kleiner als 16 Hz ist.

6. Druckwellengerät (10) nach einem der vorangehenden Ansprüche, wobei der erste Kompressor (21) und/oder der zweite Kompressor (22) konfiguriert ist, um in unterschiedlichen Leistungsstufen betreibbar zu sein, wobei die Leistungsstufen vorzugsweise stufenweise einstellbar sind.

7. Druckwellengerät (10) nach einem der vorangehenden Ansprüche, wobei das Druckwellengerät (10) eine Druckregelungseinheit (32) einschließt, die stromabwärts des ersten Kompressors (21) und/oder des zweiten Kompressors (22) und vorzugsweise stromaufwärts des Handstücks (12) angeordnet ist.

8. Druckwellengerät (10) nach Anspruch 7, wobei die Druckregelungseinheit (32) einen Druckminderer und/oder ein Ablassventil, insbesondere in Form eines Proportionalventils, einschließt.

9. Druckwellengerät (10) nach einem der vorangehenden Ansprüche, wobei hierin das Druckwellengerät (10) konfiguriert ist, um in der ersten Betriebsart umschaltbar zu sein zwischen
- einer ersten Unterbetriebsart, wobei nur der erste Kompressor (21) dem pneumatischen Antriebselement (14) Gas zuführt, und
- einer zweiten Unterbetriebsart, wobei nur der zweite Kompressor (22) dem pneumatischen Antriebselement (14) Gas zuführt.

10. Druckwellengerät nach einem der vorangehenden Ansprüche,
wobei der erste Kompressor (21) und/oder der zweite Kompressor (22) ein ölfreier Schwingkolben-Luftkompressor ist.

## Revendications

1. Dispositif à ondes de pression (10) pour le traitement des tissus biologiques, comprenant
- une pièce à main (12)
- un élément d'entraînement pneumatique (14) accélérant un projectile (28) de la pièce à main (12),
comprenant en outre
- un premier compresseur (21) et un second compresseur (22) pour fournir du gaz à l'élément d'entraînement pneumatique (14), afin d'accélérer le projectile (28),
dans lequel le dispositif à ondes de pression (10) est configuré pour pouvoir être commuté entre
-- un premier mode de fonctionnement, seul le premier compresseur (21) fournissant du gaz à l'élément d'entraînement pneumatique (14) ou seul le second compresseur (22) fournissant du gaz à l'élément d'entraînement pneumatique (14), et
-- un second mode de fonctionnement, le premier compresseur (21) et le second compresseur (22) fournissant du gaz à l'élément d'entraînement pneumatique (14).

2. Dispositif à ondes de pression (10) selon la revendication 1, comprenant en outre une unité de commande (30) pour ajuster une quantité de gaz demandée destinée à alimenter l'élément d'entraînement pneumatique (14).

3. Dispositif à ondes de pression (10) selon l'une des revendications précédentes,
dans lequel le dispositif à ondes de pression (10) est configuré pour passer du premier mode de fonctionnement au second mode de fonctionnement lorsque la quantité de gaz demandée, ajustée par l'unité de commande, dépasse une valeur seuil.

4. Dispositif à ondes de pression (10) selon l'une des revendications précédentes,
dans lequel l'unité de commande passe du premier mode de fonctionnement au second mode en se basant sur une paire de valeurs, comprenant une fréquence et une pression pour le fonctionnement du dispositif à ondes de pression (10).

5. Dispositif à ondes de pression (10) selon la revendication 4,
dans lequel le dispositif à ondes de pression (10) est configuré pour être dans le premier mode de fonctionnement, lorsque
- la fréquence demandée est inférieure à 6 Hz et/ou
- la pression demandée est inférieure à 3 bars et la fréquence demandée est inférieure à 9 Hz et/ou
- la pression demandée est inférieure à 2 bars et la fréquence demandée est inférieure à 15 Hz et/ou
- la pression demandée est inférieure à 1 bar et la fréquence demandée est inférieure à 16 Hz.

6. Dispositif à ondes de pression (10) selon l'une des revendications précédentes,
dans lequel le premier compresseur (21) et/ou le second compresseur (22) est configuré pour fonctionner à différents niveaux de puissance, les niveaux de puissance étant de préférence réglables par étapes.

7. Dispositif à ondes de pression (10) selon l'une des revendications précédentes,
dans lequel le dispositif à ondes de pression (10) comprend une unité de régulation de la pression (32) située en aval du premier compresseur (21) et/ou du second compresseur (22) et, de préférence, en amont de la pièce à main (12).

8. Dispositif à ondes de pression (10) selon la revendication 7,
dans lequel l'unité de régulation de pression (32) comprend un réducteur de pression et/ou une vanne de purge, en particulier sous la forme d'une vanne proportionnelle.

9. Dispositif à ondes de pression (10) selon l'une des revendications précédentes,
dans lequel le dispositif à ondes de pression (10) est configuré pour pouvoir être commuté, dans le premier mode de fonctionnement, entre
-- un premier sous-mode, seul le premier compresseur (21) fournissant du gaz à l'élément d'entraînement pneumatique (14) et
-- un second sous-mode, seul le second compresseur (22) fournissant du gaz à l'élément d'entraînement pneumatique (14).

10. Dispositif à ondes de pression (10) selon l'une des revendications précédentes,
dans lequel le premier compresseur (21) et/ou le second compresseur (22) est un compresseur d'air à piston oscillant exempt d'huile.
